# EUROPEAN PATENT SPECIFICATION

(11) **EP 4 203 783 B1**
(45) Date of publication and mention of the grant of the patent: **17.07.2024**
(21) Application number: 21765957.2
(22) Date of filing: 31.08.2021
(51) Int. Cl.: A61B 5/087, A61B 5/11, A61B 5/145, G16H 20/40

(54) **RESPIRATION MONITORING**
ATMUNGSÜBERWACHUNG
SURVEILLANCE DE LA RESPIRATION

(30) Priority: 31.08.2020 EP 20315396
(43) Date of publication of application: 05.07.2023
(73) Proprietor: SRETT, 92100 Boulogne-Billancourt (FR); Sorbonne Université, 75006 Paris (FR); Université Paris Cité, 75006 Paris (FR); Centre national de la recherche scientifique, 75016 Paris (FR); Institut National de la Santé et de la Recherche Médicale, 75013 Paris (FR)
(72) Inventor: ALVES PEGORARO, Juliana, 92100 Boulogne-Billancourt (FR); BIRMELE, Etienne, 76006 Paris (FR); GONZALEZ-BERMEJO, Jésus, 75006 Paris (FR); SALAMITOU, Philippe, 92100 Boulogne-Billancourt (FR); SIMILOWSKI, Thomas, 75006 Paris (FR)
(74) Representative: den Braber, Gérard Paul
(86) International application number: PCT/EP2021/074053
(87) International publication number: WO 2022/043588

(56) References cited:
- WO-A1-2005/067796
- CN-A- 111 209 816
- US-A1- 2011 112 442
- US-A1- 2017 055 877

## Description

### FIELD OF THE INVENTION

An aspect of the invention relates to a respiration monitoring processor that can provide a respiration quality indication relating to a person whose spontaneous respiration is monitored. The respiration monitoring processor may be used, for example, in deciding that a medical intervention is required in view of the respiration quality indication. Further aspects of the invention relate to a respiration monitoring system, a method carried out by a respiration monitoring processor, a method carried out by a measurement station, and a computer program.

### BACKGROUND ART

Patients with respiratory problems may suffer from acute aggravation of symptoms, exacerbations, leading to a deterioration of their state of health. The deterioration of the state of health may occur on a time scale of a day to a few days. An advanced deterioration generally entails relatively high hospital costs, reduced quality of life and increased mortality. Predicting an aggravation of respiratory problems and communicating an alert to healthcare professionals would make possible an early medical intervention, which avoids an emergency medical intervention or an irreversible deterioration of the state of health. Taken care of in time, an aggravation can be treated more effectively, by modifying a pharmacological treatment, which may involve bronchodilators, antibiotics, steroids, or non-pharmacological treatment.

Patent publication WO2015185703 describes a manner of detecting a worsening of a cardio-respiratory condition of a patient who is treated using a respiratory assistance device. A first parameter indicates a breathing rate in a first observation window. A second parameter indicates a percentage of cycles initiated by the patient during a second observation window. A third parameter indicates a duration of use of the apparatus during a third observation window. An alert is generated when a significant variation in one of these parameters is detected over at least two consecutive or nonconsecutive days during a consecutive period of at least 3 days. The alert informs the patient or a practitioner of a significant risk of worsening of the cardio-respiratory condition of the patient.

Further respiration monitoring systems and methods according to the state of the art are for instance described in CN111209816A, US2017/0055877A1, WO2005/067796A1 and US2011/0112442.

### SUMMARY OF THE INVENTION

There is a need for an improved respiration monitoring solution that allows a reliable respiration quality indication based on which an appropriate medical decision can be made.

In accordance with an aspect of the invention there is provided a respiration monitoring processor as defined in claim 1.

Such a respiration monitoring processor is able to provide a reliable respiration quality indication based on which an appropriate medical decision can be made.

In accordance with a further aspect of the invention as defined in claim 8, there is provided a respiration monitoring system. In accordance with yet further aspects of the invention, there is provided a method carried out by a respiration monitoring processor, a method carried out by a measurement station, and a computer program as defined in claims 11, 12 and 13, respectively.

For the purpose of illustration, some embodiments of the invention are described in detail with reference to accompanying drawings. In this description, additional features will be presented, some of which are defined in the dependent claims, and advantages will be apparent.

### BRIEF DESCRIPTION OF THE DRAWINGS

FIG. 1 is a block diagram of a respiration monitoring system.
FIG. 2 is a graph of an output signal of a respiration sensor in the respiration monitoring system with annotations indicating how respiration frequency may be determined.
FIG. 3 is a graph of the same output signal of the respiration sensor with annotations indicating how respiration amplitude may be determined.
FIG. 4 is a graph of a first group of measurement combinations, which indicate respiration frequency and respiration amplitude concomitantly measured on a person suffering from a chronic obstructive pulmonary disease who is in a stable condition.
FIG. 5 is a graph of a second group of measurement combinations, which indicate respiration frequency and respiration amplitude concomitantly measured on another person suffering from a chronic obstructive pulmonary disease who is an aggravated condition.
FIG. 6 is a graph of a third group of measurement combinations, which indicate respiration frequency and respiration amplitude concomitantly measured on yet another person suffering from a chronic obstructive pulmonary disease who is in a different aggravated condition.
FIG. 7 is a graph representing a set of boundaries that characterize a reference distribution applied to evaluate the second group of measurement combinations.
FIG. 8 is a graph representing the set of boundaries that characterize the reference distribution applied to evaluate the third group of measurement combinations.
FIG. 9 is a flow chart of a method carried out by a respiration monitoring processor in order to provide a respiration quality indication.

### DESCRIPTION OF SOME EMBODIMENTS

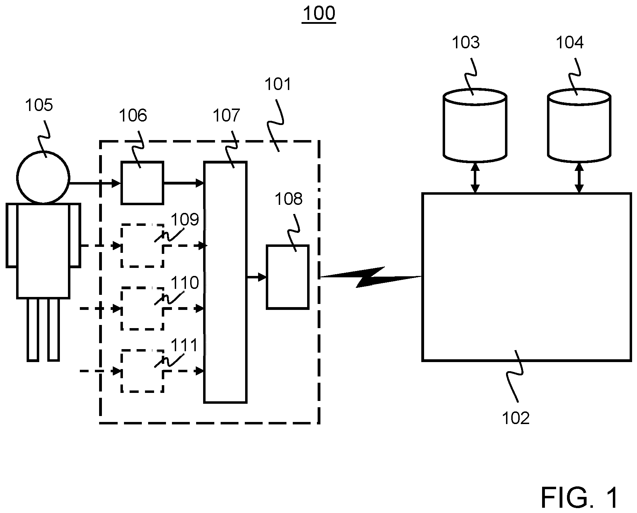

FIG. 1 schematically illustrates a respiration monitoring system 100. FIG. 1 provides a block diagram of the respiration monitoring system 100. The respiration monitoring system 100 comprises a measurement station 101, a respiration monitoring processor 102, a measurement database 103, and a medical database 104. The measurement station 101 may be located in an environment where a person 105 resides whose spontaneous respiration is to be monitored. This person will be referred to hereinafter as the monitored person 105 for the sake of convenience. The term "spontaneous" indicates that respiratory muscles primarily account for movement of gas in and out the monitored person's lungs. That is, spontaneous breathing is natural breathing.

The respiration monitoring system 100 may comprise further measurement stations for other persons whose spontaneous respiration is to be monitored. These further measurement stations are not represented in FIG. 1 for the sake of simplicity and convenience. Any further measurement station may be similar to the measurement station 101 represented in FIG. 1 and described hereinafter.

The measurement station 101 may be communicatively coupled to the respiration monitoring processor 102 through, for example, a communication network or a communication link, or a combination of these. The respiration monitoring processor 102 may comprise a server or a group of servers, or may be part of a server or a group of servers that carry out other functions. The measurement database 103, containing data from the measurement station 101, may be hosted on the same server or the same group of servers, or may be hosted on another server, or group of servers, or entity. The same applies to the medical database 104, which may contain other data from, for example, medical records.

In more detail, the measurement station 101 comprises a respiration sensor 106, a measurement processor 107, and a communication interface 108. The respiration sensor 106 may be operatively coupled to the monitored person 105 so that his or her respiration can be measured. For example, the respiration sensor 106 may measure pressure variations in an air flow path through which the person concerned may breathe. In such an embodiment, the measurement station 101 may at least partially correspond with, for example, a device as described in patent publication EP3352665. As another example, the respiration sensor 106 may comprise a nasal pressure probe, or nasal temperature probe, or a combination of these, which measure a flow of air entering and leaving the monitored person's 105 nose. As yet another example, the respiration sensor 106 may comprise one or more straps, which measure thoraco-abdominal expansion.

The measurement station 101 may further comprise at least one complementary sensor 109-111. For example, the measurement station 101 may comprise a sensor 109 that measures oxygen saturation in blood. The measurement station 101 may further comprise a sensor 110 that measures physical activity of the monitored person 105. The measurement station 101 may further comprise a capnometer 111, which measures a concentration of carbon dioxide in exhaled air.

The measurement processor 107 may comprise one or more analog to digital converters, one or more filters, and a data processor. The data processor may comprise, for example, a microprocessor and a memory containing one or more programs that the microprocessor may execute for data processing purposes and for other purposes that may be desired. These entities are not represented in FIG. 1 for the sake of simplicity and convenience.

The communication interface 108 may be, for example, of the Bluetooth type, or the LoRa type, which can provide a communication link with a communication device that provides access to a communication network, such as, for example, a mobile phone (Bluetooth is a trademark of Bluetooth SIG, Inc, and LoRa is a trademark of Semtech Corporation). Alternatively, the communication interface 108 may be, for example, of the GSM type, which can provide access to a communication network.

The respiration monitoring system 100 may basically operate as follows. By way of example, it is assumed that the respiration sensor 106 measures pressure variations in an airflow path through which the monitored person 105 breathes, Respiration of the monitored person 105 causes pressure variations in the airflow path. The pressure sensor provides an output signal that represents these pressure variations. The output signal thus conveys information on the respiration of the monitored person 105. The measurement processor 107 may filter this signal and subject the signal to an analog-to-digital conversion so as to obtain a digital version thereof that may conveniently be further processed. This processing may comprise further filtering operations, for example, to reduce noise.

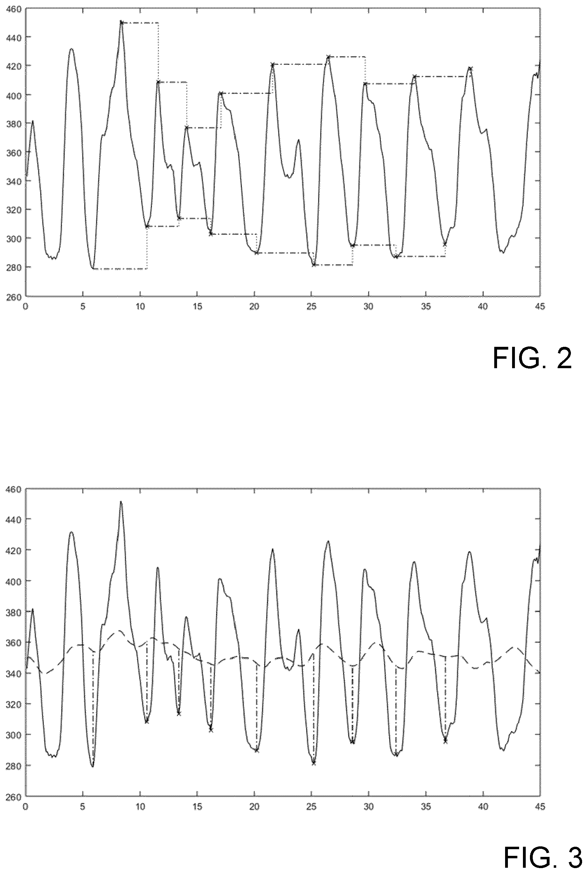

FIG. 2 schematically illustrates a filtered output signal of the respiration sensor 106 that conveys information on the respiration of the monitored person 105. FIG. 2 provides a graph that has a horizontal axis representing time and a vertical axis representing pressure. The filtered output signal is plotted in this graph, which represents measured pressure in the air flow path as a function of time.

FIG. 2 further comprises annotations indicating how the measurement processor 107 may measure a respiration frequency. The filtered output signal comprises peaks and valleys. A peak marks an end of an expiration phase of a respiratory cycle. A valley marks an end of an inspiration phase of a respiratory cycle. The annotations in FIG. 2 comprise horizontal dash-dotted lines indicating distances in time between two consecutive peaks and indicating distances in time between two consecutive valleys.

The measurement processor 107 may measure the respiration frequency in the following manner. The measurement processor 107 measures the distances in time between two consecutive peaks that occur in a time interval and the distances in time between two consecutive valleys that occur in the time interval. The measurement processor 107 may then determine a median distance in time among the aforementioned distances in time that have been measured. The inverse of the median distance in time then represents the respiration frequency in the time interval concerned.

FIG. 3 also schematically illustrates the filtered output signal of the respiration sensor 106 in a graph similar to that of FIG. 2. The graph of FIG. 3 comprises annotations indicating how the measurement processor 107 may measure a respiration amplitude. The annotations comprise a dashed reference curve with respect to which the respiration amplitude may be determined. The annotations further comprise vertical dash-dotted lines indicating distances between valleys and the reference curve.

The measurement processor 107 may measure the respiration amplitude in the following manner. The measurement processor 107 calculates a sliding average pressure, which corresponds with the dashed reference curve illustrated in FIG. 3. The measurement processor 107 then determines a pressure difference between a valley, which occurs at a certain instant, and the sliding average pressure at that instant. The measurement processor 107 may thus determine respective pressure differences for respective valleys that occur in a time interval. The measurement processor 107 may then determine a median pressure difference among these respective pressure differences. The measurement processor 107 may apply a conversion to the median pressure difference. This conversion may be based on a known relationship between measured pressure in the airflow path and a flow of air passing therethrough. The median pressure difference that is converted then represents the respiration amplitude in the time interval concerned.

The time interval in which the respiration frequency is measured as described hereinbefore and time interval in which the respiration amplitude is measured may be the same, or may at least substantially overlap. That is, the respiration frequency and the respiration amplitude are concomitantly measured. The time interval of measurement may be, for example, at least 20 seconds. The time interval may be comprised between, for example, a few tens of seconds to a few minutes. For example, in the graphs of FIGS. 2 and 3, the time interval is 45 seconds.

The respiration frequency and the respiration amplitude that are concomitantly measured constitute a measurement combination, which characterizes the monitored person's 105 respiration during a relatively short time interval. Throughout an observation period, the measurement processor 107 may thus provide respective measurement combinations based on frequency-amplitude measurements in respective time intervals, as described hereinbefore. The respective measurement combinations indicate respiration frequency and respiration amplitude of the monitored person 105 that have concomitantly been measured at respective intervals in the observation period. The observation period last be, for example, at least a few days.

The measurement processor 107 may receive and process an output signal of at least one complementary sensor. For example, the measurement processor 107 may receive and process an output signal that indicates an oxygen saturation level in the blood of the monitored person 105. As another example, the measurement processor 107 may receive and process an output signal that indicates a level of physical activity of the monitored person 105. As yet another example, the measurement processor 107 may receive and process an output signal that indicates a concentration of carbon dioxide in the air exhaled by the monitored person 105. The measurement processor 107 may thus provide complementary measurement data that represents at least one other measured physiological characteristic of the person. This complementary measurement data can be associated with the respective measurement combinations, which indicate respiration frequency and respiration amplitude.

For example, the measurement processor 107 may measure an oxygen saturation level at an instant, or in a time interval, that is within a time interval, or close thereto, in which the measurement processor 107 measures respiration frequency and respiration amplitude. The measurement processor 107 may then associate the oxygen saturation level that has been measured with the measurement combination that indicates the respiration frequency and the respiration amplitude that have been measured. There are various different manners in which such an association may be made. For example, the measurement processor 107 may put a timestamp on the oxygen saturation level that has been measured and a corresponding timestamp on the measurement combination. As another example, the measurement processor 107 may add the oxygen saturation level that has been measured to the measurement combination, which may all be incorporated in a single data packet. The aforementioned also applies to the level of physical activity and the concentration of carbon dioxide air, any of which may also be associated with the measurement combination.

The measurement station 101 may transmit measurement data to the respiration monitoring processor 102, the measurement data comprising the respective measurement combinations and the complementary measurement data, if any. Such a transmission of measurement data may be carried out through the communication interface 108. A transmission of measurement data may intermittently take place, such as, for example, on an hourly basis, or on a daily basis. The measurement processor 107 may temporarily store the measurement data to be transmitted, where after the measurement processor 107 may erase or overwrite the measurement data that has been transmitted.

The respiration monitoring processor 102 may store the measurement data that has been transmitted in the measurement database 103. The measurement data may be stored in a folder that is reserved for data concerning the monitored person 105. However, the measurement data may be associated with the monitored person 105 in the measurement database 103 in a different fashion. Accordingly, when the respiration monitoring processor 102 receives the measurement data concerning the monitored person 105, the measurement database 103 may already comprise an archive of previously received measurement data concerning that same person.

The respiration monitoring processor 102 may receive measurement data from further measurement stations, which may be similar to the measurement station 101 illustrated in FIG. 1 and described hereinbefore. The respiration monitoring processor 102 may store this measurement data, which may be similar in nature, in the measurement database 103. Thus, the measurement database 103 may comprise measurement data taken from several persons, some of which may suffer from similar diseases, or have similar symptoms, or both. The respiration monitoring processor 102 may use all of this measurement data stored in the measurement database 103 to analyze a particular group of measurement data, which will be described in greater detail hereinafter.

The respiration monitoring processor 102 may group the respective measurement combinations that have most recently been transmitted by the measurement station 101. The measurement combinations that are grouped cover a measurement period, which may correspond with, for example, one day, a fraction of the day, or several days. The respective measurement combinations that are grouped have a distribution that depends on the respiration of the monitored person 105 throughout the measurement period. This distribution of respiration frequency and respiration amplitude concomitantly measured may provide a relatively reliable indication on whether the monitored person 105 has a significant respiratory problem that needs to be treated, or not.

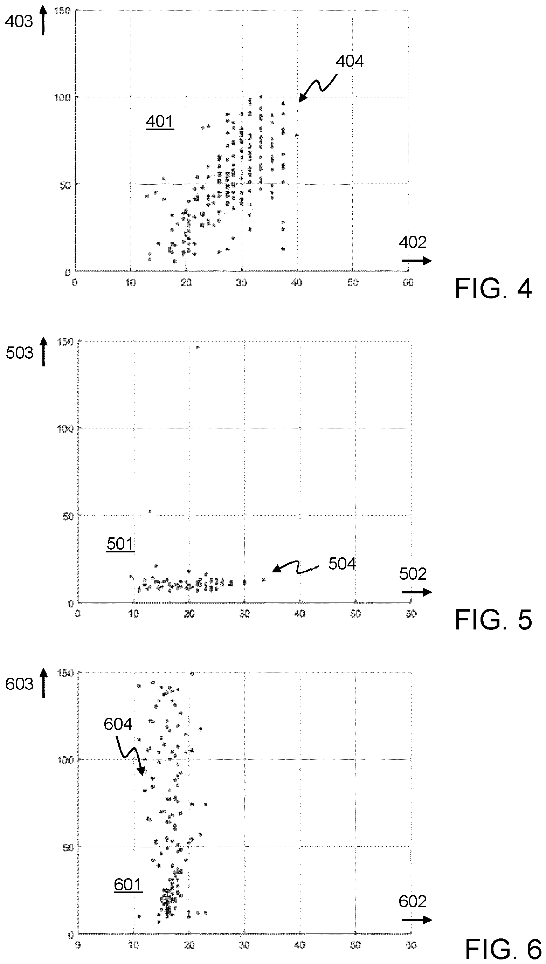

FIG. 4 schematically illustrates a first group of measurement combinations 401, which indicate respiration frequency 402 and respiration amplitude 403 concomitantly measured on a person suffering from a chronic obstructive pulmonary disease who is in a stable condition. FIG. 4 is a graph that has a horizontal axis representing the respiration frequency 402 and a vertical axis representing the respiration amplitude 403. In the graph, a measurement combination is represented as a point. The point has a horizontal position defined by the respiration frequency 402 and a vertical position defined by the respiration amplitude 403 as indicated by the measurement combination.

The first group of measurement combinations 401 has a distribution 404 that is diagonally clustered. That is, the distribution 404 can be seen as clustered along a diagonal axis extending from a point on the horizontal axis of the graph in a direction away from the horizontal axis and the vertical axis. Such a distribution 404 may characterize a healthy respiration. Namely, in cases where the respiration frequency 402 is relatively low, the respiration amplitude 403 is mostly also relatively low. This typical for a healthy person at rest. Conversely, in cases where the respiration frequency 402 is relatively high, the respiration amplitude 403 is mostly also relatively high. This is typical for a healthy person doing increased physical activity. Moreover, there is a relatively large spread in respiration frequency 402 and in respiration amplitude 403. This indicates sufficient respiratory capacity; the person is able to adapt her or his respiration to various levels of physical activity.

FIG. 5 schematically illustrates a second group of measurement combinations 501, which indicate respiration frequency 502 and respiration amplitude 503 concomitantly measured on another person suffering from a chronic obstructive pulmonary disease who is an aggravated condition. FIG. 5 is a graph similar to that of FIG. 4, except that points plotted therein are differently positioned in the graph. The points are mostly located in a bottom portion of the graph. The second group of measurement combinations 501 has a distribution 504 that is horizontally clustered in the bottom portion. The person concerned appears to have insufficient respiratory capacity due to the disease. In this case, the person's efforts to satisfy her or his respiratory needs lead to sequences of rapid and shallow breaths.

FIG. 6 schematically illustrates a third group of measurement combinations 601, which indicate respiration frequency 602 and respiration amplitude 603 concomitantly measured on yet another person suffering from a chronic obstructive pulmonary disease who is in a different aggravated condition. FIG. 6 is a graph similar to those of FIGS. 4 and 5, except that points plotted therein are differently positioned in the graph. The points are mostly located in a left-hand portion of the graph. The third group of measurement combinations 601 has a distribution 604 that is vertically clustered in the left-hand portion. The person concerned appears to have insufficient respiratory capacity due to the disease. In this case, the person's efforts to satisfy her or his respiratory needs lead to sequences of long lasting and intense breaths.

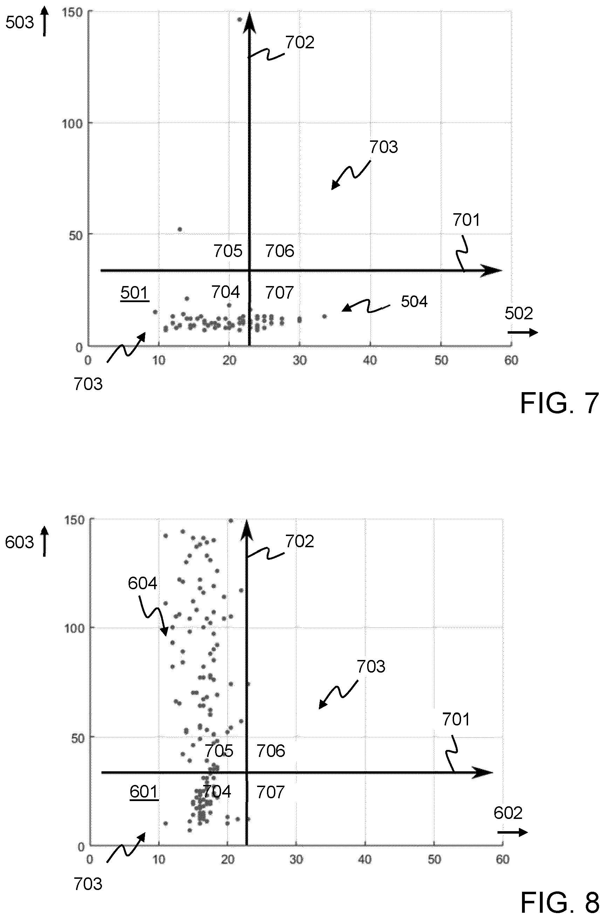

FIG. 7 schematically illustrates a set of boundaries 701, 702 characterizing a reference distribution 703 applied to evaluate the second group of measurement combinations 501 indicating respiration frequency 502 and respiration amplitude 503 concomitantly measured. FIG. 7 is a graph similar to that of FIG. 5. The graph includes a horizontal line that represents an amplitude boundary 701 of the reference distribution 703. The graph further includes a vertical line that represents a frequency boundary 702 of the reference distribution 703. The amplitude boundary 701 represented by the horizontal line and the frequency boundary 702 represented by the vertical line divide the graph into four quadrants: a lower left quadrant 704, an upper left quadrant 705, an upper right quadrant 706, and a lower right quadrant 707.

In this embodiment, the reference distribution 703 corresponds with the lower left quadrant 704 and the upper right quadrant 706, which are defined by the amplitude boundary 701 and the frequency boundary 702. A measurement combination, which is represented by a point, that is in the lower left quadrant 704 or in the upper right quadrant 706 may be considered as a "good point". Namely, such a point indicates a relatively low respiration frequency combined with a relatively low respiration amplitude, or a relatively high respiration frequency combined with a relatively high respiration amplitude, respectively. Conversely, a point representing a measurement combination that is in the upper left quadrant 705 or the lower right quadrant 707 may be considered as a "bad point". Namely, such a point indicates a relatively low respiration frequency combined with a relatively high respiration amplitude, or a relatively high respiration frequency combined with a relatively low respiration amplitude.

FIG. 8 also schematically illustrates the set of boundaries 701, 702 characterizing the reference distribution 703 applied to evaluate the third group of measurement combinations 601 indicating respiration frequency 602 and respiration amplitude 603 concomitantly measured. FIG. 8 is a graph similar to that of FIG. 7, except that points plotted therein corresponding with those plotted in the graph of FIG. 6 representing the third group of measurement combinations 601.

A reference distribution that is applied to evaluate a group of measurement combinations may be fixed or determined on the basis of data that is present in the measurement database 103, or in the medical database 104, or both. In case the reference distribution is fixed and characterized by the set of boundaries 701, 702 presented hereinbefore, the respiration amplitude boundary 701 may correspond with, for example, a pressure difference of 50 Pascal (Pa). The respiration frequency boundary 702 may correspond with, for example, 20 respirations per minute.

As another example, the reference distribution may be determined by inquiring the medical database 104 to identify several days when the monitored person 105 had a stable condition. The respiration monitoring processor 102 may then statistically analyze measurement combinations that have been provided for the monitored person 105 during these days. The respiration monitoring processor 102 may define the set of boundaries 701, 702 so that an appropriate proportion of measurement combinations are considered as "bad". This proportion should not be too high in order to avoid too pessimistic respiration quality indications, neither should the proportion be too low in order to avoid too optimistic respiration quality indications.

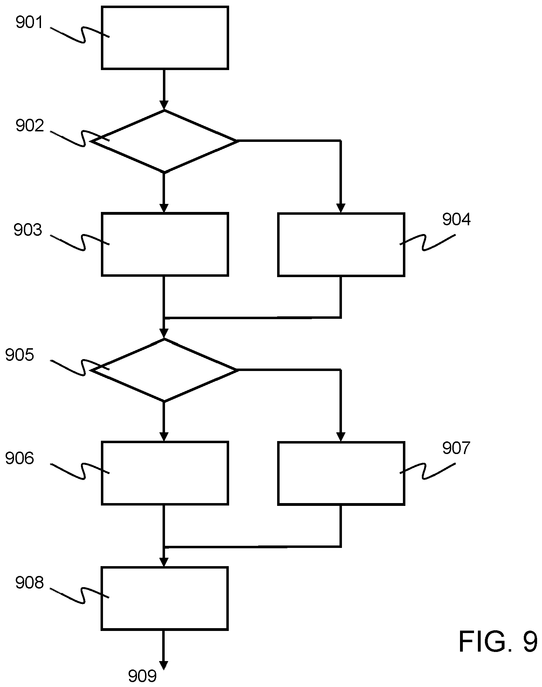

FIG. 9 schematically illustrates a method 900 that the respiration monitoring processor 102 may carry out in order to provide a respiration quality indication. FIG. 9 provides a flow chart of this method 900. The flow chart may be regarded as a schematic representation of an executable program that a computer processor, or a group of computer processors, in the respiration monitoring processor 102 may carry out. This causes the respiration monitoring processor 102 to carry out operations that will be described hereafter with reference to FIG. 9.

In a grouping step 901, the respiration monitoring processor 102 forms a group of measurement combinations that have been obtained during a measurement period, which may cover, for example, one day. The measurement combinations that form the group may, at least partially, be present in the database. The measurement combinations may also be comprised in a daily transmission of the measurement station 101. The group of measurement combinations that is formed may correspond with, for example, any of the groups 401, 501, 601 represented in the graphs of FIGS. 4-8.

In a first measurement data inspection step 902, the respiration monitoring processor 102 verifies whether the measurement data comprises indications of oxygen saturation levels are associated with measurement combinations that form the group, or not. In case this verification is negative, the respiration monitoring processor 102 carries out a basic respiration distress quantification step 903. In case this verification is positive, the respiration monitoring processor 102 carries out an advanced respiration distress quantification step 904.

In the basic distress quantification step 903, the respiration monitoring processor 102 quantifies a deviation of the measurement combinations from a reference distribution, which takes into account all the measurement combinations. The reference distribution may be characterized by a respiration amplitude boundary and a respiration frequency boundary, which defines four quadrants as described hereinbefore. In that case, the lower left quadrant and the upper right quadrant may correspond with the reference distribution. The respiration monitoring processor 102 may then determine a ratio of a number of measurements combinations that are outside the aforementioned boundaries, and thus outside the reference distribution, to a total number of measurement combinations. This ratio constitutes a metric that indicates a degree of respiratory distress in the measurement period.

In the advanced distress quantification step 904, the respiration monitoring processor 102 quantifies a deviation of the measurement combinations from the reference distribution, by taking into account only those measurement combinations that are associated with oxygen saturation levels that are below a threshold value. The threshold value may be, for example, in a range comprised between 70% and 90%. This quantification of the deviation from the reference distribution, which may be characterized by the aforementioned boundaries, will generally provide a metric that more accurately indicates a degree of respiratory distress.

Namely, a relatively low respiration frequency combined with a relatively high respiration amplitude, may due to respiratory distress, but not always. The same applies to a relatively high respiration frequency combined with a relatively low respiration amplitude. In case any of the aforementioned respiratory conditions occur, while oxygen saturation level is relatively low, this most certainly indicates respiratory distress. However, in case any of these respiratory conditions occur, while oxygen saturation level is relatively high, for example, 100%, or close thereto, these respiratory conditions are probably caused by something else than respiratory distress.

In a second measurement data inspection step 905, the respiration monitoring processor 102 verifies whether the measurement data comprises indications of levels of physical activity, or not. In case this verification is negative, the respiration monitoring processor 102 carries out a basic respiratory capacity quantification step 906. In case this verification is positive, the respiration monitoring processor 102 carries out an advanced respiratory capacity quantification step 907.

In the basic respiratory capacity quantification step 906, the respiration monitoring processor 102 quantifies respiratory capacity on the basis of respiration frequencies and on the basis of respiration amplitudes indicated by all the measurement combinations. The respiration monitoring processor 102 may calculate spread in the respiration frequencies and a spread in the respiration amplitudes, or a combined spread in frequencies and amplitudes. These spreads indicate a degree of respiratory capacity. In case these spreads are relatively wide, these indicate a relatively high degree of respiratory capacity. Conversely, in case these spreads are relatively narrow, these indicate a relatively low degree of respiratory capacity.

In the advanced respiratory capacity quantification step 907, the respiration monitoring processor 102 quantifies respiratory capacity on the basis of selected respiration frequencies and on the basis of selected respiration amplitudes. The selected respiration frequencies and the selected respiration amplitudes are those that have been measured when physical activity exceeded a threshold level. The respiration monitoring processor 102 may calculate a spread in the selected respiration frequencies and a spread in the selected respiration amplitudes, or a combined spread of these frequencies and amplitudes. These spreads based on measurements taken during physical activity will generally more accurately indicate a degree of respiratory capacity.

In a respiration quality indication generation step 908, the respiration monitoring processor 102 provides a respiration quality indication 909 on the basis of the degree of respiratory distress and the degree of respiratory capacity that have been obtained as described hereinbefore. The respiration monitoring processor 102 may further take into account data that is present in the measurement database 103, or in the medical database 104, or both, in providing the respiration quality indication.

The respiration quality indication 909 may be dichotomous, that is, binary, indicating whether the monitored person 105 is in a stable, acceptable condition, or not. The respiration quality indication 909 may also be in the form of a metric indicating, for example, a probability that the monitored person 105 is in a stable, acceptable condition, or, conversely, that the monitored person 105 has a condition that requires medical intervention. The respiration quality indication 909 may also indicate various conditions, such as, for example, a degree to which the monitored person 105 is capable of making physical efforts, a degree to which the monitored person 105 requires rest, a degree to which further medical examination is required, and a degree to which change of medication is needed. An indication of any of the aforementioned conditions may be, for example, dichotomous or a metric indicating a probability. Some embodiments of generating the respiration quality indication 909 will be presented hereinafter.

In a basic embodiment, the respiration monitoring processor 102 may provide the respiration quality indication 909 solely on the basis of the degree of respiratory distress and the degree of respiratory capacity. The degree of respiratory distress may be indicated by a metric as described hereinbefore. The degree of respiratory capacity may be indicated by a pair of metrics, indicating the spread both in the respiration frequencies, and in the respiration amplitudes. The respiration monitoring processor 102 may compare each of these metrics with a threshold value that is specific to the metric concerned. Accordingly, three dichotomous comparison results are obtained. The respiration monitoring processor 102 may then apply a logic function to these results to obtain a dichotomous respiration quality indication.

The aforementioned threshold values may be fixed, or may be determined on the basis of data that is present in the measurement database 103, or in the medical database 104, or both. For example, the aforementioned threshold values may be obtained by applying a supervised learning algorithm to the data. The data to which the supervised learning algorithm is applied may solely concern the person to be monitored. In that case, personalized threshold values are obtained and may be applied to obtain the dichotomous respiration quality indication. Alternatively, the supervised learning algorithm may be applied to all relevant data that is available in the aforementioned databases, or a selection thereof, which may thus include data concerning one or more persons other than the monitored person 105.

In an advanced embodiment, the respiration monitoring processor 102 may use data that is present in the measurement database 103 and in the medical database 104 in providing the respiration quality indication 909, in addition to the degree of respiratory distress and the degree of respiratory capacity. The respiration monitoring processor 102 may use the aforementioned data to establish a function that receives the degree of respiratory distress and the degree of respiratory capacity as inputs and, in response thereto, provides the respiratory quality indication.

The respiration monitoring processor 102 may establish the function by a learning process that is applied to the data that is present in the measurement database 103 and in the medical database 104. This learning process may be based on a supervised learning algorithm. This may involve, for example, training a neural network, a random forest, or logistic regressions, or a combination of these. The training may be based on archived measurement data, which may be annotated using the medical database 104. The training may be based on data concerning several persons, indicating occurrence of clinical episodes, such as, for example, exacerbations. In the advanced embodiment, the respiratory quality indication may thus express a probability of the monitored person 105 being in a condition that requires medical intervention.

As another example, the learning process may be of the one-class classification-type. Such a process estimates a probability that a new point belongs to a cloud of reference points for the monitored person 105. This process may be based on, for example, a mahalanobis distance-based method. For this method, only the data concerning the monitored person 105 is used.

Complementary, the respiration monitoring processor 102 may classify the monitored person 105, as well as other persons, in a category corresponding to her or his medical care needs. Typical profiles may be learned from archived measurement data using an unsupervised classification technique, or a clustering technique, such as moving averages or ascending hierarchical classification. These profiles correspond, for example, to groups of persons whose measurement combinations have similar distributions. That is, the distributions are similar within a group and different between groups. A group may require a specific form of medical care, whereas another group may require a different specific form of medical care.

### NOTES

The embodiments described hereinbefore with reference to the drawings are presented by way of illustration. The disclosure may be implemented in numerous different ways. In order to illustrate this, some alternatives are briefly indicated.

The disclosure may be applied in numerous types of products or methods related to respiration monitoring.

There are numerous different ways of measuring respiration frequency and respiration amplitude in a product or method in accordance with the disclosure.

In the presented embodiment, a specific algorithm is applied to determine respiration frequency and respiration amplitude from an output signal of a respiration sensor. Other algorithms may be applied. For example, an algorithm may be based on an analysis of a Fourier decomposition of the signal. It is also possible to use an adaptative algorithm based on an auto regressive model.

In the presented embodiment, there is a specific repartition of processing tasks among the respiration sensor 106, the measurement processor 107, the communication interface 108 and the respiration monitoring processor 102. All these entities may have processing and communication capabilities. Thus, the repartition of processing tasks among these entities can be modified. For example, in an alternative embodiment, the respiration monitoring processor 102 may carry out substantially all processing tasks, whereas, the measurement station 101 merely transmits raw measurement data to the respiration monitoring processor 102. In another alternative embodiment, some, or even all, of the processing tasks that are carried out by the measurement processor 107 in the presented embodiment, may be carried out by the communication interface 108. In yet another alternative embodiment, at least a part of the processing tasks carried out by the respiration monitoring processor 102 in the presented embodiment, may be carried out by a processor in the measurement station 101. In such an embodiment, any information that this processor may require to carry out such a processing task, may be transmitted from respiration monitoring processor 102 to the measurement station 101.

There are numerous different ways of characterizing a reference distribution in accordance with the invention. In the presented embodiment, the reference distributions are defined by a horizontal line that represents an amplitude boundary and a vertical line that represents a frequency boundary. In other embodiments, the reference distributions may be defined by one or more continuous probability distributions, for instance a gaussian distribution, from which probabilities of each measurement combination being a "good point" or "bad point" can be computed.

Furthermore, measurement data inspection steps presented hereinbefore may be carried out in different orders or not be performed at all. Metrics described hereinbefore for quantification of distress and respiratory capacity are an example of implementation. In other embodiments, deviations from the reference distribution may be quantified as a distance between the measurement combination points and a reference probability distribution or as a likelihood of observing measurement combination points given the reference probability distribution.

In general, there are numerous different ways of implementing the invention, whereby different implementations may have different topologies. In any given topology, a single entity may carry out several functions, or several entities may jointly carry out a single function. In this respect, the drawings are very diagrammatic. For example, respiration monitoring in accordance with the invention may at least partially be carried out by various computing resources in a distributed manner, which is generally referred to as "cloud computing". Furthermore, there are numerous functions that may be implemented by means of hardware or software, or a combination of both. A description of a software-based implementation does not exclude a hardware-based implementation, and vice versa. Hybrid implementations, which comprise one or more dedicated circuits as well as one or more suitably programmed processors, are also possible. For example, various functions described hereinbefore with reference to the figures may be implemented by means of one or more dedicated circuits, whereby a particular circuit topology defines a particular function.

There are numerous ways of storing and distributing a set of instructions, that is, software, which allows respiration monitoring in accordance with the invention. For example, software may be stored in a suitable device readable medium, such as, for example, a memory circuit, a magnetic disk, or an optical disk. A device readable medium in which software is stored may be supplied as an individual product or together with another product, which may execute the software. Such a medium may also be part of a product that enables software to be executed. Software may also be distributed via communication networks, which may be wired, wireless, or hybrid. For example, software may be distributed via the Internet. Software may be made available for download by means of a server. Downloading may be subject to a payment.

The remarks made hereinbefore demonstrate that the embodiments described with reference to the drawings illustrate the invention, rather than limit the invention. The invention can be implemented in numerous alternative ways that are within the scope of the appended claims. Any reference sign in a claim should not be construed as limiting the claim. The verb "comprise" in a claim does not exclude the presence of other elements or other steps than those listed in the claim. The same applies to similar verbs such as "include" and "contain". The mention of an element in singular in a claim pertaining to a product, does not exclude that the product may comprise a plurality of such elements. Likewise, the mention of a step in singular in a claim pertaining to a method does not exclude that the method may comprise a plurality of such steps. The mere fact that respective dependent claims define respective additional features, does not exclude combinations of additional features other than those reflected in the claims.

## Claims

1. A respiration monitoring processor (102) adapted to receive respective measurement combinations (401, 501, 601) indicating at least respiration frequency (402, 502, 602) and respiration amplitude (403, 503, 603) of a spontaneously breathing person (105) that have concomitantly been measured at respective intervals throughout an observation period,
the respiration monitoring processor being adapted to form a group of measurement combinations covering a measurement period of at least several hours, the measurement combinations in the group having a distribution (404, 504, 604) that depends on the person's respiration throughout the measurement period,
the respiration monitoring processor being adapted to analyze the group of measurement combinations covering the measurement period of at least several hours by:
- quantifying a deviation of the measurement combinations in the group from a reference distribution (703), the deviation indicating a degree of respiratory distress in the measurement period, and
- calculating a frequency spread and an amplitude spread in the group of measurement combinations, said spreads indicating a degree of respiratory capacity,
the respiration monitoring processor being adapted to provide a respiration quality indication (909) based on the degree of respiratory distress indicated by the deviation from the reference distribution and on the degree of respiratory capacity indicated by the frequency spread and the amplitude spread.

2. A respiration monitoring processor according to claim 1, wherein the respiration monitoring processor is adapted to define the reference distribution (703) on the basis of previously received measurement combinations concerning the person (105).

3. A respiration monitoring processor according to any of claims 1 and 2, wherein the respiration monitoring processor is adapted to define the reference distribution (703) on the basis of data from a medical file of the person (105).

4. A respiration monitoring processor according to any of claims 1 to 3, wherein respiration monitoring processor is adapted to define the reference distribution (703) on the basis of previously received measurement combinations concerning a group of persons.

5. A respiration monitoring processor according to any of claims 1 to 4, wherein the respiration monitoring processor further receives respective measurements of oxygen saturation level in the blood of the person (105) in association with the measurement combinations (401, 501, 601), the respiration monitoring processor being adapted to take into account only measurement combinations associated with measurements of oxygen saturation level that are below a threshold value in determining the deviation of the measurement combinations in the group from the reference distribution (703).

6. A respiration monitoring processor according to any of claims 1 to 5, wherein the reference distribution (703) is **characterized by** a set of boundaries (701, 702), the respiration monitoring processor being adapted to provide the respiration quality indication (909) based on a proportion of the measurement combinations (401, 501, 601) in the group that are outside the set of boundaries.

7. A respiration monitoring processor according to any of claims 1 to 6, wherein the respiration monitoring processor further receives respective indications of level of physical activity by the person in association with the respective measurement combinations (401, 501, 601), the respiration monitoring processor being adapted to take into account only measurement combinations associated with indications of level of physical activity that exceed a threshold in determining at least one of the following: the frequency spread in the measurement combinations in the group and the amplitude spread in the measurement combinations in the group.

8. A respiration monitoring system (100) comprising:
- a measurement station (101) adapted to provide respective measurement combinations (401, 501, 601) representing at least respiration frequency (402, 502, 602) and respiration amplitude (403, 503, 603) of a spontaneously breathing person (105) that have concomitantly been measured at respective intervals throughout an observation period; and
- a respiration monitoring processor (102) according to any of claims 1 to 7, which is adapted to process the respective measurement combinations provided by the measurement station so as to provide the respiration quality indication (909).

9. A respiration monitoring system according to claim 8, wherein the respective intervals are time intervals of at least 20 seconds.

10. A respiration monitoring system according to any of claims 8 and 9, wherein the measurement station (101) is adapted to provide complementary data that represents at least one other measured physiological characteristic of the person (105), and wherein the respiration monitoring processor (102) is adapted to take into account the complementary data in providing the respiration quality indication (909).

11. A method carried out by a respiration monitoring processor (102) that executes a computer program, the method comprising:
- receiving respective measurement combinations (401, 501, 601) representing at least respiration frequency and respiration amplitude of a spontaneously breathing person (105) that have concomitantly been measured at respective intervals throughout an observation period,
- forming (901) a group of measurement combinations covering a measurement period of at least several hours, the measurement combinations in the group having a distribution (404, 504, 604, 703) that depends on the person's respiration throughout the measurement period,
- analyzing the group of measurement combinations covering the measurement period of at least several hours by:
- quantifying (903) a deviation of the measurement combinations in the group from a reference distribution (703), the deviation indicating a degree of respiratory distress in the measurement period,
- calculating (906) a frequency spread and an amplitude spread in the group of measurement combinations, said spreads indicating a degree of respiratory capacity, and
- providing (908) a respiration quality indication (909) based on the degree of respiratory distress indicated by the deviation from the reference distribution and on the degree of respiratory capacity indicated by the frequency spread and the amplitude spread.

12. A method carried out by a measurement station (101), the method comprising:
- concomitantly measuring at least respiration frequency and respiration amplitude of a spontaneously breathing person at respective intervals throughout a measurement period of at least several hours; and
- transmitting measurement combinations (401, 501, 601) to a respiration monitoring processor (102) according to any of claims 1 to 7, the measurement combinations being received by said respiration monitoring processor and representing at least the respiration frequency and respiration amplitude of the person that have concomitantly been measured.

13. A computer program that enables a processor to carry out a method according to claim 11.

## Patentansprüche

1. Atmungsüberwachungsprozessor (102), der dazu ausgebildet ist, jeweilige Messkombinationen (401, 501, 601) zu empfangen, die zumindest die Atemfrequenz (402, 502, 602) und die Atemamplitude (403, 503, 603) einer spontan atmenden Person (105) angeben, die während eines Beobachtungszeitraums in jeweiligen Abständen gleichzeitig gemessen wurden,
wobei der Atmungsüberwachungsprozessor so angepasst ist, dass er eine Gruppe von Messkombinationen bildet die einen Messzeitraum von mindestens mehreren Stunden abdeckt, wobei die Messkombinationen in der Gruppe eine Verteilung (404, 504, 604) aufweisen, die von der Atmung der Person während des Messzeitraums abhängt,
wobei der Atmungsüberwachungsprozessor so angepasst ist, dass er die Gruppe von Messkombinationen die einen Messzeitraum von mindestens mehreren Stunden abdeckt analysiert durch:
- Quantifizieren einer Abweichung der Messkombinationen in der Gruppe von einer Referenzverteilung (703), wobei die Abweichung einen Grad an Atemnot im Messzeitraum angibt, und
- Berechnen einer Frequenzstreuung und einer Amplitudenstreuung in der Gruppe von Messkombinationen, wobei die Streuungen einen Grad der Atemkapazität angeben,
wobei der Atmungsüberwachungsprozessor dazu ausgebildet ist, eine Anzeige der Atmungsqualität (909) basierend auf dem durch die Abweichung von der Referenzverteilung angezeigten Grad der Atemnot und auf dem durch die Frequenzstreuung und die Amplitudenstreuung angezeigten Grad der Atemkapazität bereitzustellen.

2. Atmungsüberwachungsprozessor gemäß Anspruch 1, wobei der Atmungsüberwachungsprozessor dazu geeignet ist, die Referenzverteilung (703) auf der Grundlage zuvor empfangener Messkombinationen bezüglich der Person (105) zu definieren.

3. Atmungsüberwachungsprozessor gemäß einem der Ansprüche 1 und 2, wobei der Atmungsüberwachungsprozessor dazu geeignet ist, die Referenzverteilung (703) auf der Grundlage von Daten aus einer Krankenakte der Person (105) zu definieren.

4. Atmungsüberwachungsprozessor gemäß einem der Ansprüche 1 bis 3, wobei der Atmungsüberwachungsprozessor dazu geeignet ist, die Referenzverteilung (703) auf der Grundlage zuvor empfangener Messkombinationen bezüglich einer Gruppe von Personen zu definieren .

5. Atmungsüberwachungsprozessor nach einem der Ansprüche 1 bis 4, wobei der Atmungsüberwachungsprozessor außerdem jeweilige Messungen des Sauerstoffsättigungsgrads im Blut der Person (105) in Verbindung mit den Messkombinationen (401, 501, 601) empfängt, wobei der Atmungsüberwachungsprozessor dazu ausgebildet ist, bei der Ermittlung der Abweichung der Messkombinationen in der Gruppe von der Referenzverteilung (703) nur Messkombinationen zu berücksichtigen, die mit Messungen des Sauerstoffsättigungsgrads verbunden sind, die unter einem Schwellenwert liegen.

6. Atmungsüberwachungsprozessor gemäß einem der Ansprüche 1 bis 5, wobei die Referenzverteilung (703) durch eine Reihe von Grenzen (701, 702) gekennzeichnet ist, wobei der Atmungsüberwachungsprozessor dazu ausgebildet ist, die Atmungsqualitätsangabe (909) basierend auf einem Anteil der Messkombinationen (401, 501, 601) in der Gruppe bereitzustellen, die außerhalb der Reihe von Grenzen liegen.

7. Atmungsüberwachungsprozessor gemäß einem der Ansprüche 1 bis 6, wobei der Atmungsüberwachungsprozessor außerdem jeweilige Angaben zum Niveau körperlicher Aktivität der Person in Verbindung mit den jeweiligen Messkombinationen (401, 501, 601) empfängt, wobei der Atmungsüberwachungsprozessor dazu ausgebildet ist, bei der Bestimmung von mindestens einem der folgenden Elemente nur Messkombinationen zu berücksichtigen, die mit Angaben zum Niveau körperlicher Aktivität verbunden sind, die einen Schwellenwert überschreiten: die Frequenzstreuung in den Messkombinationen in der Gruppe und die Amplitudenstreuung in den Messkombinationen in der Gruppe.

8. Atmungsüberwachungssystem (100), umfassend:
- eine Messstation (101), die dazu ausgebildet ist, jeweilige Messkombinationen (401, 501, 601) bereitzustellen, die zumindest die Atemfrequenz (402, 502, 602) und die Atemamplitude (403, 503, 603) einer spontan atmenden Person (105) darstellen, die während eines Beobachtungszeitraums in jeweiligen Abständen gleichzeitig gemessen wurden; und
- einen Atmungsüberwachungsprozessor (102) gemäß einem der Ansprüche 1 bis 7, der dazu ausgebildet ist, die jeweiligen von der Messstation bereitgestellten Messkombinationen zu verarbeiten, um die Atmungsqualitätsanzeige (909) bereitzustellen.

9. Atmungsüberwachungssystem nach Anspruch 8, wobei die jeweiligen Intervalle Zeitintervalle von mindestens 20 Sekunden sind.

10. Atmungsüberwachungssystem nach einem der Ansprüche 8 und 9, wobei die Messstation (101) dazu ausgebildet ist, ergänzende Daten bereitzustellen, die mindestens eine weitere gemessene physiologische Eigenschaft der Person (105) darstellen, und wobei der Atmungsüberwachungsprozessor (102) dazu ausgebildet ist, die ergänzenden Daten bei der Bereitstellung der Atmungsqualitätsanzeige (909) zu berücksichtigen.

11. Verfahren, das von einem Atmungsüberwachungsprozessor (102) ausgeführt wird, der ein Computerprogramm ausführt, wobei das Verfahren umfasst:
- Empfangen von jeweiligen Messkombinationen (401, 501, 601), die zumindest die Atemfrequenz und die Atemamplitude einer spontan atmenden Person (105) repräsentieren, die während eines Beobachtungszeitraums in jeweiligen Zeitabständen gleichzeitig gemessen wurden,
- Bilden (901) einer Gruppe von Messkombinationen, die einen Messzeitraum von mindestens mehreren Stunden abdecken, wobei die Messkombinationen in der Gruppe eine Verteilung (404, 504, 604, 703) aufweisen, die von der Atmung der Person während des Messzeitraums abhängt,
- Analyse der Gruppe der Messkombinationen Abdeckung des mindestens mehrere Stunden umfassenden Messzeitraums durch:
- Quantifizieren (903) einer Abweichung der Messkombinationen in der Gruppe von einer Referenzverteilung (703), wobei die Abweichung einen Grad der Atemnot im Messzeitraum angibt,
- Berechnen (906) einer Frequenzstreuung und einer Amplitudenstreuung in der Gruppe von Messkombinationen, wobei die Streuungen einen Grad der Atemkapazität angeben, und
- Bereitstellen (908) einer Atmungsqualitätsangabe (909) auf der Grundlage der Grad der Atemnot der durch die Abweichung von der Referenzverteilung angezeigt wird und der Grad der Atemkapazität der durch die Frequenzstreuung und die Amplitudenstreuung angezeigt wird.

12. Von einer Messstation (101) durchgeführtes Verfahren, wobei das Verfahren umfasst:
- gleichzeitiges Messen von zumindest der Atemfrequenz und der Atemamplitude einer spontan atmenden Person in entsprechenden Zeitabständen über einen Messzeitraum von mindestens mehreren Stunden; und
- Übertragen von Messkombinationen (401, 501, 601) an einen Atmungsüberwachungsprozessor (102) gemäß einem der Ansprüche 1 bis 7, wobei die Messkombinationen von dem Atmungsüberwachungsprozessor empfangen werden und zumindest die gleichzeitig gemessene Atemfrequenz und Atemamplitude der Person darstellen.

13. Computerprogramm, das es einem Prozessor ermöglicht, ein Verfahren gemäß Anspruch 11 auszuführen.

## Revendications

1. Processeur de surveillance respiratoire (102) adapté pour recevoir des combinaisons de mesures respectives (401, 501, 601) indiquant au moins la fréquence respiratoire (402, 502, 602) et l'amplitude respiratoire (403, 503, 603) d'une personne respirant spontanément ( 105) qui ont été mesurées concomitamment à des intervalles respectifs tout au long d'une période d'observation ,
le processeur de surveillance respiratoire étant adapté pour former un groupe de combinaisons de mesures couvrant une période de mesure d'au moins plusieurs heures, les combinaisons de mesures du groupe ayant une répartition (404, 504, 604) qui dépend de la respiration de la personne pendant toute la période de mesure,
le processeur de surveillance respiratoire étant adapté pour analyser le groupe de combinaisons de mesures couvrant la période de mesure d'au moins plusieurs heures par :
- quantifier un écart des combinaisons de mesures dans le groupe par rapport à une distribution de référence (703), l'écart indiquant un degré de détresse respiratoire dans la période de mesure, et
- calculer un étalement de fréquence et un étalement d'amplitude dans le groupe de combinaisons de mesures, lesdits étalements indiquant un degré de capacité respiratoire,
le processeur de surveillance respiratoire étant adapté pour fournir une indication de qualité respiratoire (909) basée sur le degré de détresse respiratoire indiqué par l'écart par rapport à la distribution de référence et sur le degré de capacité respiratoire indiqué par l'étalement de fréquence et l'étalement d'amplitude.

2. Processeur de surveillance respiratoire selon la revendication 1, dans lequel le processeur de surveillance respiratoire est adapté pour définir la distribution de référence (703) sur la base de combinaisons de mesures reçues précédemment concernant la personne (105).

3. Processeur de surveillance respiratoire selon l'une quelconque des revendications 1 et 2, dans lequel le processeur de surveillance respiratoire est adapté pour définir la distribution de référence (703) sur la base de données provenant d'un dossier médical de la personne (105).

4. Processeur de surveillance respiratoire selon l'une quelconque des revendications 1 à 3, dans lequel le processeur de surveillance respiratoire est adapté pour définir la distribution de référence (703) sur la base de combinaisons de mesures reçues précédemment concernant un groupe de personnes.

5. Processeur de surveillance respiratoire selon l'une quelconque des revendications 1 à 4, dans lequel le processeur de surveillance respiratoire reçoit en outre des mesures respectives du niveau de saturation en oxygène dans le sang de la personne (105) en association avec les combinaisons de mesures (401, 501, 601), le processeur de surveillance respiratoire étant adapté pour prendre en compte uniquement les combinaisons de mesures associées aux mesures du niveau de saturation en oxygène qui sont inférieures à une valeur seuil pour déterminer l'écart des combinaisons de mesures dans le groupe par rapport à la distribution de référence (703).

6. Processeur de surveillance respiratoire selon l'une quelconque des revendications 1 à 5, dans lequel la distribution de référence (703) est **caractérisée par** un ensemble de limites (701, 702), le processeur de surveillance respiratoire étant adapté pour fournir l'indication de qualité respiratoire (909). sur la base d'une proportion des combinaisons de mesures (401, 501, 601) dans le groupe qui sont en dehors de l'ensemble de limites.

7. Processeur de surveillance respiratoire selon l'une quelconque des revendications 1 à 6, dans lequel le processeur de surveillance respiratoire reçoit en outre des indications respectives du niveau d'activité physique de la personne en association avec les combinaisons de mesures respectives (401, 501, 601), le processeur de surveillance respiratoire étant adapté pour prendre en compte uniquement les combinaisons de mesures associées à des indications de niveau d'activité physique qui dépassent un seuil pour déterminer au moins l'un des éléments suivants : l'étalement de fréquence dans les combinaisons de mesures dans le groupe et l'étalement d'amplitude dans les combinaisons de mesures dans le groupe.

8. Système de surveillance respiratoire (100) comprenant :
- une station de mesure (101) adaptée pour fournir des combinaisons de mesures respectives (401, 501, 601) représentant au moins la fréquence respiratoire (402, 502, 602) et l'amplitude respiratoire (403, 503, 603) d'une personne respirant spontanément (105). qui ont été mesurés concomitamment à des intervalles respectifs tout au long d'une période d'observation ; et
- un processeur de surveillance respiratoire (102) selon l'une quelconque des revendications 1 à 7, qui est adapté pour traiter les combinaisons de mesures respectives fournies par la station de mesure de manière à fournir l'indication de qualité respiratoire (909).

9. Système de surveillance respiratoire selon la revendication 8, dans lequel les intervalles respectifs sont des intervalles de temps d'au moins 20 secondes.

10. Système de surveillance respiratoire selon l'une quelconque des revendications 8 et 9, dans lequel la station de mesure (101) est adaptée pour fournir des données complémentaires qui représentent au moins une autre caractéristique physiologique mesurée de la personne (105), et dans lequel le processeur de surveillance respiratoire (102) est adapté pour prendre en compte les données complémentaires en fournissant l'indication de qualité respiratoire (909).

11. Procédé mis en oeuvre par un processeur de surveillance respiratoire (102) qui exécute un programme informatique, le procédé comprenant :
- recevoir des combinaisons de mesures respectives (401, 501, 601) représentant au moins la fréquence respiratoire et l'amplitude respiratoire d'une personne respirant spontanément (105) qui ont été mesurées simultanément à des intervalles respectifs tout au long d'une période d'observation,
- former (901) un groupe de combinaisons de mesures couvrant une période de mesure d'au moins plusieurs heures, les combinaisons de mesures du groupe ayant une répartition (404, 504, 604, 703) qui dépend de la respiration de la personne pendant toute la période de mesure,
- analyser le groupe de combinaisons de mesures couvrant la période de mesure d'au moins plusieurs heures par :
- quantifier (903) un écart des combinaisons de mesures dans le groupe par rapport à une distribution de référence (703), l'écart indiquant un degré de détresse respiratoire dans la période de mesure,
- calculer (906) un étalement de fréquence et un étalement d'amplitude dans le groupe de combinaisons de mesures, lesdites étalements indiquant un degré de capacité respiratoire, et
- fournir (908) une indication de qualité respiratoire (909) basée sur la degré de détresse respiratoire indiqué par l'écart par rapport à la distribution de référence et sur le degré de capacité respiratoire indiqué par l'étalement de fréquence et l'étalement d'amplitude.

12. Procédé réalisé par une station de mesure (101), le procédé comprenant :
- mesurer simultanément au moins la fréquence respiratoire et l'amplitude respiratoire d'une personne respirant spontanément à des intervalles respectifs tout au long d'une période de mesure d'au moins plusieurs heures ; et
- transmettre des combinaisons de mesures (401, 501, 601) à un processeur de surveillance respiratoire (102) selon l'une quelconque des revendications 1 à 7, les combinaisons de mesures étant reçues par ledit processeur de surveillance respiratoire et représentant au moins la fréquence respiratoire et l'amplitude respiratoire du personne qui ont été mesurées simultanément.

13. Programme informatique permettant à un processeur de mettre en oeuvre un procédé selon la revendication 11.
